Europäisches Patentamt

European Patent Office

Office européen des brevets

(11) Publication number: **0 344 166 B1**

# EUROPEAN PATENT SPECIFICATION

(45) Date of publication of patent specification: **16.09.92**   (51) Int. Cl.5: **G01N 33/569**, G01N 33/545

(21) Application number: **88900561.7**

(22) Date of filing: **23.12.87**

(86) International application number:
**PCT/AU87/00439**

(87) International publication number:
**WO 88/04778 (30.06.88 88/14)**

(54) **DIAGNOSTIC TEST FOR SWINE DYSENTERY.**

(30) Priority: **23.12.86 AU 9631/86**

(43) Date of publication of application:
**06.12.89 Bulletin 89/49**

(45) Publication of the grant of the patent:
**16.09.92 Bulletin 92/38**

(84) Designated Contracting States:
**AT BE CH DE FR GB IT LI LU NL SE**

(56) References cited:
**EP-A- 242 082**
**WO-A-86/02354**
**US-A- 4 418 152**

**BIOLOGICAL ABSTRACTS, vol. 74, no. 2, 1982, Biological Abstracts Inc., Philadelphia, PA (US); L.A.JOENS et al., no. 10942**

**JOURNAL OF IMMUNOLOGICAL METHODS, vol. 85, 1985, Elsevier Science Publishers BV, Amsterdam (NL); J.G.KENNA et al., pp. 409-419**

**CHEMICAL ABSTRACTS, vol. 100, no. 11, March 12, 1984, Columbus, OH (US); A.V.GOLIKOV et al., p. 289, no. 82611v**

**CHEMICAL ABSTRACTS, vol. 106, no. 5, February 2, 1987, Columbus, OH (US); L.A.JOENS et al., p. 378, no. 31066n**

(73) Proprietor: **ROYAL MELBOURNE INSTITUTE OF TECHNOLOGY LIMITED**
**124 La Trobe Street**
**Melbourne, VIC 3000(AU)**

(72) Inventor: **COLOE, Peter, John**
**23 Tidcombe Crescent**
**East Doncaster, VIC 3109(AU)**

(74) Representative: **Holmes, Michael John et al**
**Frank B. Dehn & Co. European Patent Attorneys Imperial House 15-19 Kingsway**
**London, WC2B 6UZ,(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a diagnostic test kit and method which may be used to determine the degree of immunity of pigs to swine dysentery, for example as a result of prior exposure of the pigs to infection or after vaccination.

Treponema hyodysenteriae has been identified as the primary aetiological agent of swine dysentery. It is accepted within the veterinary profession that pigs which have recovered from swine dysentery develop a degree of immunity to the disease. However, if the animals have been treated with antibiotics during the course of the disease, their resistance to subsequent infection is lessened. This suggests that for adequate resistance to infection to develop, the animals must be subjected to extensive, long term exposure to the causative agent, Treponema hyodysenteriae. Since T.hyodysenteriae manifests its disease effects at the mucosal surface of the large intestine this implies that, for immunity to develop, active clinical infection must occur.

Relatively little is known about the type of immune response of pigs to T.hyodysenteriae infection and no data is available as to the type of antibody response (i.e., IgG, IgM, IgA) elicited by exposure to T.hyodysenteriae.

Enzyme linked immunosorbent assays (ELISA) have been developed for many infectious diseases in man and animals. However, no ELISA or other serological tests have been developed to date for the reliable detection of swine dysentery in pigs. An ELISA for detection of antibody to certain lipopolysaccharide (LPS) antigens of T.hyodysenteriae has been reported by Joens et.al. in J. Clin. Microbiol. 15(2), 249-252, 1982 but has the limitation of being serotype specific since the antigens concerned are only a fraction of the total antigens on the whole cells. Fernie et.al. Research in Veterinary Science (1983) 35, 217-221, have reported a microagglutination test for determining the antibody response to T.hyodysenteriae infection, but they could not determine any correlation between the magnitude of the titres they determined and a degree of protection to experimental challenge. Glock et.al., U.S. Patent No. 4,100,272 have determined antibody titres against T.hyodysenteriae, using an indirect fluorescent antibody technique, however no commercially acceptable test has resulted from this technique.

The present invention provides an effective, reliable and more generally applicable, serological diagnostic test method for swine dysentery using, for example, the ELISA technique, together with a diagnostic test kit to enable performance of this test.

According to a first aspect of this invention, there is provided a diagnostic test method for the detection of antibodies against Treponema hyodysenteriae in a sample taken from a pig, which comprises the steps of bringing said sample into contact with T.hyodysenteriae antigen on a solid support, and detecting binding of antibodies against T.hyodysenteriae in said sample to said antigen, characterised in that said antigen is whole cell antigen, in that the antigen on the solid support has been treated with a solution of casein or other milk protein to prevent non-specific binding prior to said sample being brought into contact with said antigen and in that said antibody binding is detected using labelled anti-porcine antibody.

In another aspect, this invention provides a diagnostic test kit for the detection of antibodies against T.hyodysenteriae in a sample taken from a pig, which comprises:

    i. T.hyodysenteriae whole cell antigen on a solid support;

    ii. a solution of casein or other milk protein; and

    iii. means including labelled anti-porcine antibody for the detection of binding of antibodies against T.hyodysenteriae in a sample to said antigen.

Preferably, the "blocking" step to prevent non-specific binding is carried out using a solution of non-fat skim milk in phosphate buffer. Other solutions used in the test method, for example, enzyme/antibody conjugate solutions and wash solutions preferably also contain skim milk in phosphate buffer. The use of casein as a blocking agent in immunoassays is discussed generally by Kenna et.al. in J. Immunol. Methods 85, 409-419, 1985 but no mention is made of its utility in a T.hyodysenteriae specific assay.

Preferably, the means for the detection of binding of the antibodies to said antigen on the solid support comprises anti-porcine IgG or IgM having an enzyme, such as horseradish peroxidase, conjugated thereto, together with a chromogenic substrate such as ABTS which reacts with the enzyme to provide a detectable signal.

The following detailed description sets out the steps of the assay method of this invention:

    1. Cells of Treponema hyodysenteriae, are grown in a fermenter or on agar plates, harvested washed with (PBS) phosphate buffered saline, sonicated and stored at -20°C until required for use as antigen.

    2. The T.hyodysenteriae antigen is added to a solid support such as a Nunc high-bond polystyrene ELISA microtitre plate and stored overnight at 4°C for the antigen to bind to the surface of the wells of the plastic support.

    3. Any unbound antigen is removed from the support surface, and a solution of non-fat skim milk (5% in PBS) is added to coat any free receptor sites on the support surface that might otherwise bind serum proteins.

4. Pig serum, diluted to the appropriate dilutions (for example from 1/400 to 1/51200) is added to the wells and allowed to react with the T.hyodysenteriae antigen.

5. After reaction, the pig serum is washed off and affinity-purified antiporcine IgG, or affinity-purified antiporcine IgM, both of which are conjugated to an indictor enzyme, are added to the wells. (The conjugate solutions are preferably diluted in PBS with 2% non-fat skim milk).

6. After reaction, the wells are again washed with PBS-2% non-fat skim milk and then a substrate that reacts with the enzyme-conjugated IgG or IgM is added to the wells.

7. The extent of binding of the enzyme-conjugated antiserum is determined using appropriate techniques.

8. In each assay, sera from a pig with a known antibody titre to T.hyodysenteriae (positive control) and a serum from a pig with no previous exposure (negative control) are included.

The ELISA assay for T.hyodysenteriae in pigs provided by the present invention has been used to determine the antibody response in pigs that have been vaccinated with a T.hyodysenteriae vaccine, or have been naturally infected. When pigs are challenged with a virulent wild-type strain of T.hyodysenteriae, it is found that those that have an absorbance titre of >1.0 at a dilution of 1/800 for IgG are resistant to experimental or natural infection with T.hyodysenteriae, whereas those with an absorbance of less than 1.0 at a serum dilution of 1/800 are susceptible to natural or experimental infection. (It is found that all pigs that have not previously been exposed to T.hyodysenteriae have absorbance levels of less than 0.5 at 1/800).

Further features of the present invention will be apparent from the following Examples.

EXAMPLE 1

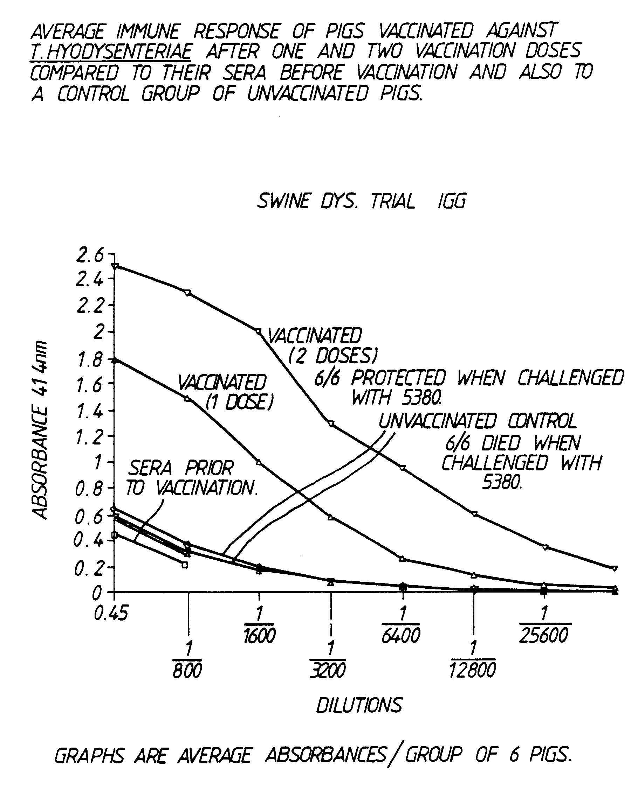

The assay procedure of the present invention was used to determine the average immune response of pigs vaccinated against T.hyodysenteriae after 1 and 2 vaccination doses compared to their sera before vaccination, and also compared to a control group of unvaccinated pigs. The results are shown in the accompanying figure. All of these pigs (vaccinates and controls) were challenged with a virulent strain of T.hyodysenteriae and all control pigs died of classical swine dysentery whereas none of the vaccinates showed any signs of swine dysentery. From these results, it has been concluded that an absorbance of 1.0 at a 1/800 dilution of serum is the minimum antibody titre required to give protection against T.hyodysenteriae infection.

EXAMPLE 2

The ELISA assay of this invention has been applied to a number of herds of varying status to swine dysentery. In 3 individual herds under the same management but on isolated properties, the swine dysentery status was determined using this T.hyodysenteriae ELISA assay.

**Herd 1a** Status - Transient cases of swine dysentery in recent weeks. 16/20 pigs with T.hyodysenteriae titres.

**b**. ELISA results confirmed T.hyodysenteriae infection.

**Herd 2a** Status - No evidence of Swine dysentery, but some pigs from herd 1 had been introduced 2 months earlier.

**b** ELISA results confirmed T.hyodysenteriae infection present in 20% pigs. The herd subsequently broke with cases of clinical swine dysentery 2 weeks later.

**Herd 3a** Status - No evidence of swine dysentery in recent years and no introduction of new pigs in recent months. 0/20 pigs with T.hyodysenteriae titres.

**b** ELISA results indicated no evidence of T.hyodysenteriae infection. Herd still free of swine dysentery.

**Claims**

1. A diagnostic test method for the detection of antibodies against Treponema hyodysenteriae in a sample taken from a pig, which comprises the steps of bringing said sample into contact with T.hyodysenteriae antigen on a solid support, and detecting binding of antibodies against T.hyodysenteriae in said sample to said antigen, characterised in that said antigen is whole cell antigen, in that the antigen on the solid support has been treated with a solution of casein or other milk protein to prevent non-specific binding prior to said sample being brought into contact with said antigen and in that said antibody binding is detected using labelled anti-porcine antibody.

2. A method according to claim 1, wherein said solution used to prevent non-specific binding comprises a solution of non-fat skim milk in phosphate buffer.

3. A method according to claim 1 or claim 2, wherein said detecting step comprises the step of addition of anti-porcine antibody having an enzyme conjugated thereto, followed by a chromogenic substrate for said enzyme to provide a detectable signal.

4. A method according to claim 3, wherein said enzyme-conjugated anti-porcine antibody is horseradish peroxidase-conjugated anti-porcine IgG or IgM, and said chromogenic substrate is ABTS.

5. A diagnostic test kit for use in the test method of claim 1 which comprises:
   i. T.hyodysenteriae whole-cell antigen on a solid support;
   ii. a solution of casein or other milk protein; and
   iii. means including labelled anti-porcine antibody for the detection of binding of antibodies against T.hyodysenteriae in a sample to said antigen.

6. A test kit according to claim 5, wherein said solution of casein or other milk protein comprises a solution of non-fat skim milk in phosphate buffer.

7. A test kit according to claim 5 or claim 6, wherein said detection means comprises anti-porcine antibody having an enzyme conjugated thereto, and a chromogenic substrate for said enzyme.

8. A test kit according to claim 7, wherein said enzyme-conjugated anti-porcine antibody is horseradish peroxidase-conjugated anti-porcine IgG or IgM, and said chromogenic substrate is ABTS.

**Patentansprüche**

1. Diagnostisches Testverfahren zur Detektion von Antikörpern gegen Treponema hyodysenteriae in einer einem Schwein entnommenen Probe, wobei man
   die Probe mit T. hyodysenteriae-Antigen auf einem festen Träger in Kontakt bringt und
   die Bindung von Antikörpern gegen T. hyodysenteriae in der Probe an das Antigen detektiert,
   **dadurch gekennzeichnet**, daß das Antigen ein Ganzzellen-Antigen ist, das Antigen auf dem festen Träger mit einer Lösung aus Casein oder einem anderen Milchprotein zur Verhinderung von unspezifischer Bindung behandelt wird, bevor die Probe mit dem Antigen in Kontakt gebracht wird und daß die Antikörperbindung unter Verwendung eines markierten Anti-Porcin-Antikörpers detektiert wird.

2. Verfahren nach Anspruch 1, worin die Lösung, welche zur Vermeidung von unspezifischer Bindung verwendet wird, eine Lösung aus entfetteter Magermilch in Phosphatpuffer umfaßt.

3. Verfahren nach Anspruch 1 oder Anspruch 2, worin der Detektionsschritt die Zugabe von Anti-Porcin-Antikörper mit einem daran ankunjugierten Enzym, gefolgt von einem chromogenen Substrat für das Enzym zur Bereitstellung eines detektierbaren Signals umfaßt.

4. Verfahren nach Anspruch 3, worin der enzymkonjugierte Anti-Porcin-Antikörper Meerrettichperoxidase-konjugiertes Anti-Porcin-IgG oder -IgM ist, und das chromogene Substrat ABTS ist.

5. Diagnostischer Testkit zur Verwendung in dem Testverfahren gemäß Anspruch 1, umfassend:
   i. T. hyodysenteriae-Ganzzellenantigen auf einem festen Träger;
   ii. eine Lösung aus Casein oder einem anderen Milchprotein; und
   iii. Mittel, enthaltend markierte Anti-Porcin-Antikörper zur Detektion der Bindung von Antikörpern gegen T. hyodysenteriae in der Probe an das Antigen.

6. Testkit gemäß Anspruch 5, worin die Lösung aus Casein oder einem anderen Milchprotein eine Lösung aus entfetteter Magermilch in Phosphatpuffer umfaßt.

7. Testkit nach Anspruch 5 oder Anspruch 6, worin das Mittel zur Detektion Anti-Porcin-Antikörper mit einem daran ankonjugierten Enzym und ein chromogenes Substrat für das Enzym umfaßt.

8. Testkit nach Anspruch 7, worin der Enzym-konjugierte Anti-Porcin-Antikörper Meerrettichperoxidase-konjugiertes Anti-Porcin-IgG oder -IgM und das chromogene Substrat ABTS ist.

**Revendications**

1. Procédé de test diagnostique pour la détection d'anticorps contre Treponema hyodysenteriae dans un échantillon prélevé sur un porc, qui comprend les étapes consistant à mettre l'échantillon précité en contact avec un antigène de T. hyodysentiriae sur un support solide et à déceler la liaison d'anticorps contre T.hyodysenteriae dans ledit échantillon à de l'antigène précité, caractérisé en ce que cet antigène est un antigène de cellule entière ou complète, en ce que l'antigène sur le support solide a été traité par une solution de caséine, ou d'une autre protéine de lait, afin d'empêcher une liaison aspécifique préalablement à la mise en contact de l'échantillon concerné avec l'antigène en cause et en ce que ladite liaison de l'anticorps est décelée en utilisant un anticorps antiporcin marqué.

2. Procédé suivant la revendication 1, caractérisé en ce que la solution utilisée pour empêcher la liaison aspécifique est constituée par une solution de lait écrémé non gras dans un tampon au phosphate.

3. Procédé suivant la revendication 1 ou la revendication 2, caractérisé en ce que l'étape de détection comprend le stade d'addition d'anticorps antiporcin auquel une enzyme est conjuguée, suivie de celle d'un substrat chromogène pour l'enzyme concernée de façon à donner un signal décelable.

4. Procédé suivant la revendication 3, caractérisé en ce que l'anticorps antiporcin conjugué à l'enzyme concernée est l'IgM ou l'IgG antiporcine conjuguée à de la raifort-peroxydase et le substrat chromogène précité est l'ABTS.

5. Trousse pour test diagnostique à utiliser pour la mise en oeuvre du procédé de test suivant la revendication 1, caractérisée en ce qu'elle comprend :
   i. de l'antigène de cellule entière de T. hyodysenteriae sur un support solide,
   ii. une solution de caséine, ou d'une autre protéine de lait et
   iii. des moyens comprenant de l'anticorps antiporcin marqué pour la détection de la liaison de l'anticorps contre T. hyodysenteriae dans un échantillon à l'antigène précité.

6. Trousse de test selon la revendication 5, caractérisée en ce que la solution de caséine, ou d'une autre protéine de lait, est constituée d'une solution de lait écréme non gras dans un tampon au phosphate.

7. Trousse d'essai selon la revendication 5 ou la revendication 6, caractérisée en ce que le moyen de détection comprend un anticorps antiporcin auquel est conjuguée une enzyme et un substrat chromogène pour l'enzyme concernée.

8. Trousse de test suivant la revendication 7, caractérisée en ce que l'anticorps antiporcin conjugué à l'enzyme est de l'IgM ou de l'IgG antiporcine conjuguée à de la raifort-peroxydase et le substrat chromogène précité est l'ABTs.

AVERAGE IMMUNE RESPONSE OF PIGS VACCINATED AGAINST
T.HYODYSENTERIAE AFTER ONE AND TWO VACCINATION DOSES
COMPARED TO THEIR SERA BEFORE VACCINATION AND ALSO TO
A CONTROL GROUP OF UNVACCINATED PIGS.

SWINE DYS. TRIAL   IGG

GRAPHS ARE AVERAGE ABSORBANCES/GROUP OF 6 PIGS.